# EUROPEAN PATENT APPLICATION

(11) **EP 4 610 365 A1**
(43) Date of publication of application: **03.09.2025**
(21) Application number: 24160210.1
(22) Date of filing: 28.02.2024
(51) Int. Cl.: C12Q 1/6804, G01N 33/50

(54) **SINGLE MOLECULE DETECTION SYSTEM AND METHOD**

(71) Applicant: Mugenbio GmbH, 8908 Hedingen (CH)
(72) Inventor: Jozwiakowski, Stanislaw, 8908 Hedingen (CH)
(74) Representative: P&TS SA (AG, Ltd.)

(57) **Abstract**

This invention concerns a molecular probe comprising an identifier biopolymer conjugated with a binding molecule able to bind to a target analyte, wherein the identifier biopolymer comprises one or more structural elements having a defined nanostructure, said nanostructure being defined by the primary sequence of the identifier biopolymer, wherein said structural element is configured to generate a unique identification signal when translocating through a nanopore sensor. The invention further concerns a method and a system for detecting and/or quantifying a target analyte in a sample, which uses this molecular probe.

## Description

### Technical domain

The present invention concerns the detection and quantification of single analytes in a sample.

### Related art

The human genome contains approximately 20'000 to 24'000 protein coding genes. However, due to various epigenetic regulations, different RNA splicing and post translational modification, the actual size of human proteome is estimated to exceed one million of different protein variants. This exceedingly large number of protein variants is prerequisite for building diversity of human cell types and multitude of biological processes taking place in these biological compartments. This high degree of variability provides an enormous potential for research and medical applications.

Proteome diversity and dynamics characterizes "healthy" cells and changes in response to various internal and external stimuli. Distinct changes in the proteome can also be observed in "unhealthy" cells providing unique molecular signatures / molecular fingerprints, of human diseases.

Accurate and highly sensitive detection and quantification of proteins and their variants is therefore fundamental for our detailed understanding of natural biological processes. Moreover, it holds the key for enhanced medical diagnostics and personalised treatments.

Given the size and complexity of the human proteome, there is a need for protein detection method which is characterized with ultra-sensitive, preferably single molecule detection resolution. Ideally the detection should be easily multiplexable to at least 50 or at least 100 analytes at the time, such as to enable a comprehensive and rapid analysis of a limited sample volume.

However, protein detection and quantification methods available today are not capable to meet all the above requirements. Current methods include widely used techniques such as Mass Spectrometry (MS) and traditional immunoassays like RadioimmunoAssay (RIA), Enzyme-Linked ImmunoSorbent Assay (ELISA), Fluorescence ImmunoAssay (FIA), and ChemiLuminescence ImmunoAssay (CLIA).

While MS can quantify numerous proteins simultaneously, it is limited by high abundance requirements and expensive equipment. Traditional immunoassays are sensitive but are often restricted to single analyte detection and have calibration curve dependencies.

To overcome limitations, bead array-based immunoassays were introduced, allowing multiplexed analysis. However, these methods have sensitivity constraints and are not suitable for high-multiplex scenarios. Nucleic acid-based immunoassays, like immuno-PCR, Proximity Extension Assay (PEA), Proximity Ligation Assay (PLA), and Nucleic acid Linked Immuno-Sandwich Assay (NULISA), offer high multiplexing capabilities but are limited by sensitivity. In addition, these methods do not directly measure the presence or quantity of an analyte but rely on a quantitative polymerase chain reaction (qPCR) amplification step, thereby reducing the accuracy and reliability of the measurement. Recent improvements involve pairing PEA and NULISA with Next Generation Sequencing (NGS), yet the cost and instability of biological nanopore sensors pose challenges. Moreover, the NGS sequencing step is time intense and too expensive to be used in routine diagnostic tests.

Despite the considerable advances in molecular technology and diagnostic tools over the past decades a reliable, cost- and time-efficient detection and/or quantification method capable of detecting single analytes in a highly multiplexed assay still remains elusive today.

### Short disclosure of the invention

It is an aim of this invention to provide a method and system for detecting target analytes, preferably target molecules, in a sample which overcome the drawbacks of current state of the art methods.

It is another aim of this invention to provide a method and system for detecting target analytes which is highly-sensitive and suitable for accurate quantification of the target analyte. The method and system should be capable of detecting single molecules of target analytes.

It is another aim of this invention to provide a method and system for detecting target analytes which can be performed rapidly and at low cost, such that they are suitable for everyday clinical diagnosis and personalised treatments.

It is yet another aim of the present invention to provide a method and system for detecting single analytes in a solution and which can be used in highly-multiplexed assays capable of detecting at least 50 or more different target analytes.

According to the invention, these aims are attained by the object of the attached claims, and especially by the independent claims. The dependent claims provide optional embodiments of the claimed invention.

In particular, one or more of the above-stated aims are attained by a molecular probe comprising an identifier biopolymer conjugated with a binding molecule able to bind to a target analyte, preferably a target molecule, wherein the identifier biopolymer comprises one or more structural elements having a defined nanostructure. The nanostructure of the structural elements is defined by the primary sequence of the identifier biopolymer. The unique identifier biopolymer serves as a specific identity label of the binding molecule.

The one or more structural element may be formed in the identifier biomolecule itself. The structural element may also be formed by the biomolecule and modifying elements, such as additional nucleotide strands hybridized with portions of a nucleotide biomolecule.

The one or more structural element may be stabilized by inter- and/or intra-molecule covalent bonds.

The structural element is configured such as to generate a unique identification signal when translocating through a pore of a nanopore sensor.

In a preferred embodiment the nanopore sensor is a solid-state nanopore sensor, comprising nanopores fabricated from synthetic material, such as SiO₂, SiN, HfOz, Al₂O₃, ZnO, TiOz, BN, MoS₂, WS₂, MXenes, polymerised polyurea, graphene, glass and/or quartz. Compared with biological nanopores, solid-state nanopores provide the advantage of mechanical robustness and durability. In addition, the pore size and geometry are readily tunable, thereby offering greater flexibility for specific assay needs.

The identifier bio-polymer provides a unique barcode nanostructure. This unique nanostructure generates a unique identification signal which is detectable by a nanopore sensor.

Since the identifier biopolymer is conjugated with a binding molecule designed to bind a specific target analyte, the binding specificity of each molecular probe is inherently linked to its identification signal provided by the identifier biopolymer. The "identifier" biopolymer is hereinafter also referred to as a "barcode" biopolymer, a "barcode molecule, or simply as a "barcode".

The nanostructure or a structural element is preferably defined by the monomeric sequence of the portion of the biopolymer, which forms the structural element. In other words, each structural element self-assembles into a nanostructure which is defined by the primary sequence of the biopolymer.

The one or more structural elements preferably have a cross-sectional dimension which is larger than the largest cross-sectional dimension of the other portions of the identifier biopolymer. In other words, as determined by their cross-section, the structural elements are the largest portions of the identifier biopolymer.

The one or more structural elements may be positively or negatively charged compared to the other portions of biopolymer.

The hydrophobicity of the one or more structural elements may be higher or lower compared to the other portions of biopolymer.

The one or more structural elements may have a different thermal stability which may be higher or lower compared to the other portions of biopolymer.

The one or more structural elements may have a different mechanical flexibility which may be higher or lower compared to the portions of biopolymer.

The one or more structural element may comprise modifications, for example functional groups or other elements linked to the one or more structural elements, which introduce the above mentioned altered chemical and/or physical properties to the one or more structural element to distinguish it from the other portions of the biopolymer.

To permit its use in nanopore sensors, the structural element dimensioned to be able to translocate a nanopore. The largest cross-sectional dimension of the structural element should therefore not be bigger than the size of a nanopore. Generally, a nanopore is a pore of nanometre size. Typical ranges or the pores in nanopore sensors range from 1nm to 200 nm, or from 1.5nm to 100nm or from 2nm to 50nm, or from 3 to 30nm. Accordingly, the largest cross-sectional dimension of the structural element should be smaller than the largest nanopore used in the nanopore sensor to allow for translocation. The largest cross-sectional dimension of the structural element may for example range from 0.5nm to 180nm, or from 1nm to 90nm, 1.5nm to 45nm, or 2nm to 30nm.

In one embodiment of this invention the identifier biopolymer is or comprises a polynucleotide molecule, preferably a synthetic polynucleotide molecule.

In one embodiment of this invention the identifier biopolymer is or comprises a synthetic polynucleotide molecule selected from a group consisting of a single strand desoxyribonucleic acid (DNA), a double strand DNA, a single strand xeno nucleic acid (XNA), a double strand XNA, or a combination thereof. The polynucleotide may for example be a homopolymeric double strand or it may be an at least partially heteropolymeric double strand, for example a DNA/XNA double strand, or it may be an at least partially heteropolymeric triple strand, for example a XNA/DNA/XNA triple strand. An identifier nucleotide polymer may for example have a length ranging from 20nm to 4000nm, or form 30nm to 3000nm, or from 35nm to 2000nm, or 40 to 1000nm. A double strand nucleotide polymer may for example comprise from 100 bp to 10 kbp.

The biopolymer of this invention is preferably a linear polymer. It is however also possible that the biopolymer is a branched or a networked biopolymer, as long as it is suitable for translocation through a nanopore of a nanopore sensor.

In one embodiment, the single unique structural element positioned in a central portion of the identifier biopolymer serves as the center point of reference within the biopolymer. The central positioning of the structural element provides advantage that the identifier can be read the same way bi-directionally.

In one embodiment the identifier biopolymer is formed mirror-symmetrically to its transverse median plane. In other words, the two halves on either side of the central point of the longitudinal axis of the identifier biopolymer are mirror symmetrical to one another. The identifier polymer may be a coded in a palindromic manner. Mirror symmetrical biopolymers provide the advantage that the identifier biopolymer can be read bi-directionally. The unique pattern providing the digital identifier is the same when read in either direction, akin to a palindromic pattern. This means that the signal caused by the passage of the biopolymer through the nanopore is independent from which of the two ends of the biopolymer enters the nanopore first. The unique identifier biopolymer will result in the same electrical signal no matter in which of its two orientation it enters the nanopore. This is particularly useful in assays in which identifier biopolymers are separated from the molecular probe before entering the nanopore.

A palindromic coding pattern permits for easy confirmation of the integrity of the identifier biopolymer by comparing the corresponding portions of the palindromic sequence to one another.

The translocation velocity and orientation of the biopolymer sequence during translocation can be controlled by the number and distribution of uncharged, or positive, or negative monomeric units over the length of the identifier biopolymer. Negatively charged portions may be used to guide the polymer through the pore of a nanopore sensor or to increase the translocation velocity. Uncharged or positively charged monomeric units on the other hand may be used to slower down translocation of the biopolymer.

In one embodiment the biopolymer is provided with a negatively charged portion at a free end, i.e. an end which is not conjugated with the binding molecule. This negative portion serves as a polyanionic leader element. Due to its negative charge this polyanionic leader element guides the identifier biopolymer through the pore of a nanopore sensor.

Negative charges may for example be altered by a XNA modification, for example PNA- peptide nucleic acid, HNA- 1,5-nhydrohexitol nucleic acid, CeNa- cyclohexane nucleic acid, TNA- therose nucleic acid, GNA- glycol nucleic acid, or FANA- fluoroarabino nucleic acid, of a DNA polymer. The use of the DNA analogues with uncharged backbone opens possibility of finetuning the polyanionic character of our DNA barcodes.

The PNA for example be provided as a patch in the polymeric DNA molecule, thereby modulating its polyanionic character. A PNA portion can thus serve as portion with decreased negative charge as well as a modulator of the translocation velocity of the polymeric DNA *via* the nanopore.

Portions of PNA in an DNA polymer also affect the equilibrium of self-assembly of the structural elements, as PNA may be used to modulate the melting temperature of the DNA secondary structures. PNA portions or PNA patches can therefore be used to modulate the physical properties, for example thermal stability and mechanical flexibility, of the structural elements.

Additionally, or alternatively the translocation velocity of an identifier molecule may be modulated by providing hydrophobic portions or modifications, which can modulate interaction with the surface of nanopore channel and can potentially modulate the speed of translocation of the identifier molecule.

The identifier biopolymer may be coupled to the binding molecule through a conjugation element with thiol group or amine group or azide group or alkynyl group, which can create a stable covalent link between the identifier biopolymer and the binding molecule. The conjugation element is chosen according to the type of biopolymer and the type of binding molecule.

For example, the conjugation of a binding molecule which is a polypeptide and an identifier biopolymer, which is a DNA barcode molecule can be realized by positioning a conjugation element at the 3' end and/or at the 5' end of the DNA barcode molecule. The conjugation of the binding molecule with the 3' end and/or 5' end of DNA barcode can be achieved via disulfide linkage, amide bond or azide-alkyne cycloaddition, also known as the Click chemistry.

It is however also possible to position a conjugation element at the junction between two identical identifier biopolymers, such that the binding molecule is coupled at the junction of two joined identifier biopolymers. The resulting molecular probe comprises two barcode twin arms, i.e. two identical biopolymers. Providing two identical barcode arms on the same molecular probe allows bi-directional reading of the unique identification signal of the molecular probe capturing the analyte and measured by the nanopore sensor.

In an embodiment where the barcode two arms are DNA molecules the conjugation element and/or binding molecule may be attached to a phosphate, sugar and/or nitrogen base moiety or any other functional group of a modified or an xeno nucleic acid analogue inserted between the twin DNA barcodes.

A suitable conjugation element may also be used to facilitating covalent linkage and/or non-covalent method of immobilization of identifier biopolymer to the solid phase support. Suitable conjugation elements for this purpose are characterised with thiol group or amine group or azide group or alkynyl group or biotin or avidin or digoxigenin or fluorophore, for example fluorescein.

In one embodiment the identifier biopolymer comprises cleavable elements, for example sequences specific for restriction endonucleases in a dsDNA polymer, or photo-cleavable linkers in ssDNA at its terminal ends, which allow for a separation of the identifier biopolymer from any covalently molecule linked to its ends, for example the binding molecule, and/or for removing an identifier biomolecule from a substrate to which it is attached. By means of the cleavable elements, such as sequences specific for restriction endonucleases in a dsDNA polymer, or UV sensitive photo-cleavable linkers, a free identifier biopolymer can readily be retrieved. This is particularly useful if the nanopore sensor assay is designed to detect and/or quantify target analytes based on the translocation of the identifier biopolymer only, rather than on translocation of the entire molecular probe bound to target analyte.

The identifier biopolymer may be further functionalized with a hapten molecule, for example by covalently linking a small molecule, such as biotin, or digoxigenin, or 2,4-dinotrophenyl (DNP) or a small molecule fluorescent dye, for example fluorescein, to the biopolymer.

Additionally, the identifier biopolymer may be further functionalised by introduction of internal covalent bonds stabilising the structural features and increasing mechanical rigidity of the biopolymer molecule. For example, such functionalisation of the identifier biopolymer can be achieved via intra- and inter-strand covalent bonds formation using the Click chemistry.

In one embodiment the binding molecule of the molecular probe has a molecular mass of 1kDa to 100kDa or of 2kDa to 80kDa or of 5kDa to 60kDa.

The binding molecule may be selected from a group existing of a single-chain variable fragment (scFv), a camelid (VHH), a small protein binder, such as a monobody, an affibody, an anticalin, a DARPin, or a nucleic acid-based protein binder, such as a DNA aptamer or an RNA aptamer.

The invention also concerns a method for the detecting and/or quantifying a target analyte in a sample using the above-described molecular probe and a nanopore sensor. By combining the high degree of sensitivity of the nanopore sensor with the unique barcode nanostructure of the biopolymer, the method is configured for single molecule resolution.

Since the method does not need any amplification steps to increase the amount of DNA barcode molecule for measurement with PCR amplification or quantify the amount of the DNA barcode with qPCR quantification, the measurements are direct, more reliable and more accurate than methods relying on additional amplification of the biological marker of DNA barcode.

Moreover, the method does not require any sequencing steps to read the primary barcode sequence, as the identification information is provided by the secondary structure of the barcode, i.e. by the nanostructure of the one or more structural elements of the identifier biopolymer.

The unique nanostructure of each identifier biopolymer generates a specific electrical identification signature when it passes through a pore of the nanopore sensor.

To determine the presence of quantity of a target analyte in a sample, the molecular probe is incubated with a sample which potentially contains the target analyte. The incubation is performed under suitable conditions, i.e. conditions which permit for binding of a target analyte to the binding molecule of the molecular probe. Incubation conditions are defined according to the analyte and binding molecule. Similarly, the incubation time is sufficiently long to permit binding of the target analyte to the binding molecule.

Optionally, following the incubation, molecular probes which have not bound a target molecule may be removed.

The molecular probes which have bound the target analyte are then analysed using a nanopore sensor. For this purpose the molecular probes are translocated through the pores of the sensor, thereby generating a unique identification signal which is specific to the barcode of the molecular probe, i.e. its identifier polymer.

Alternatively, the unique identifier biopolymer may be separated from the molecular probe and passed through the nanopore sensor without the remainder of the molecular probe. Since the molecular probes which have not bound the target analyte were removed in a prior step, only identifier biopolymers of molecules which have bound the target analyte are measured with the nanopore sensor.

Based on the unique identification signal generated during translocation of the identifier biopolymer, respectively the molecular probe, a statement is made about the presence and/or the quantity of a target analyte in the analysed sample. The translocation of the identifier biopolymer through the nanopore, as part of the molecular probe or separately, causes an alteration of a current signal in the nanopore sensor. The altered current signal is specific for the translocated unique identifier biopolymer.

The assay method described herein may be performed to detect and/or quantify one specific target analyte.

The assay is also suitable to detect a plurality of different target analytes in a sample. For this purpose a plurality of corresponding molecular probe, each comprising a binding molecule for one specific target analyte of the plurality of analytes and at least one unique identifier biopolymer which is conjugated to said specific binding molecule. The assay of this invention is therefore suitable for multiplexing, allowing to detect a large number of different target analytes in one assay. This results in significant savings in time and labour for the analysis of samples containing many different target analytes, without having to compromise on the molecular resolution of the assay. In fact, multiplexing the assay does not result in loss of detection sensitivity. In addition, a multiplexed analysis is also more cost-efficient than sequential analysis of the sample.

The number of different target molecules which can be detected and/or quantified using this multiplexed method is not particularly limited as thousands of unique ssDNA and dsDNA barcodes can be assembled / coded.

In one embodiment 2 to 200, 2 to 100, or 2 to 50 different target analytes may be detected and/or quantified in the same assay.

In one embodiment of this invention the molecular probe is immobilised on a solid support during the incubation step. In a subsequent step either the molecular probe is removed from the support or the identifier biopolymer is separated from the molecular probe. The released molecular probe or the separated identifier biopolymer may then be applied to the nanopore sensor for measurement.

This invention further concerns a system comprising the molecular probe with the identifier biopolymer described herein and a nanopore sensor.

With respect to what is known in the art, the invention provides the advantage that it combines the highly specific capture of the protein analytes with the single molecule counting capability of the solid state nanopore sensors. The bridging of these two approaches is achieved by development of molecular probes containing specific binding molecules, for example small proteins, and modular nanostructure barcodes, i.e. identifier biopolymers such as nucleic acid identifiers. In essence, the nanostructure barcodes and the molecular probes are engineered to be readable and distinguishable by the solid state nanopore sensors. Ultimately, the novel method for protein detection and quantification has huge potential to fulfil the current needs and unlock the progress of cost-effective and ultra-sensitive high multiplex proteomics and *in vitro* diagnostics.

### DEFINITIONS

The term "nanopore sensor" refers to a through-hole, referred to as a "nanopore" in a membrane, said nanopore having a largest cross-sectional dimension, for example a diameter, as small as 1 nm, or as small as 2nm, combined with a means of electronical or combination of electronical and optical detection of single molecules passing through said nanopore.

The term "biopolymer" as used herein refers to a biological polymer comprising 50 monomeric units or more. For example, a dsDNA bio-polymer comprises at least 50 bp. The term biopolymer includes polynucleotides, such as DNA, XNA, for example PNA.

The term "target analyte" refers to the analyte that the molecular probe is designed to bind, respectively that the system of this invention is designed to detect and quantify.

The target analyte may be a biological molecule. It may also be a chemical compound.

The singular "a", "an" and "the" include the plural equivalents unless the context distinctly indicates otherwise.

The terms "comprises" and "contains mean "includes" in a non-limiting sense.

The terms "e.g.", "for example" and "such as" as used herein indicate specific non-limiting examples that fall under a more general category.

The singular "a", "an" and "the" include the plural equivalents unless the context distinctly indicates otherwise.

The terms "comprises" and "contains" mean "includes" in a non-limiting sense.

The terms "e.g.", "for example" and "such as" as used herein indicate specific non-limiting examples that fall under a more general category.

### Short description of the drawings

Exemplar embodiments of the invention are disclosed in the description and illustrated by the drawings in which:
Figure 1 illustrates schematically possible embodiments of molecular probes according to this invention,
Figure 1A illustrates a molecular probe with one barcode arm, or identifier biopolymer, and
Figure 1B illustrates a molecular probe with twin barcode arms,
Figure 2 illustrates schematically examples for nucleic acid nanostructure motifs which may be used in an identifier biopolymer, wherein
Figure 2A shows a hairpin motif,
Figure 2B shows a G4 quadruplex motif,
Figure 2C shows a 3'-5' Flap dsDNA patch,
Figure 2D shows a dsDNA patch,
Figure 2E shows a ssPNA patch,
Figure 2F shows a PNA-DNA-PNA triple helix motif, and
Figure 2G shows a nano-molecule tethering nanostructure;
Figure 3 illustrates schematically examples nanostructure motifs based on dsDNA, which may be used in an identifier biopolymer, wherein
Figure 3A shows a cruciform motif,
Figure 3B shows ds hairpin motif,
Figure 3C shows loop motif,
Figure 3D shows a dsDNA G4 quadruplex motif,
Figure 3E shows a ds 3'-5' flap motif,
Figure 3F shows a ds 5'-3' flap motif,
Figure 3G shows a ds gap motif,
Figure 3H shows a ds gap motif,
Figure 3I shows a dsPNA patch,
Figure 3J shows a PNA-DNA-PNA triple helix loop motif, and
Figure 3K shows a ds nano-molecule tethering nanostructure;
Figure 4 shows a modular assembly of exemplar DNA barcodes of this invention, wherein
Figure 4A shows a modular assembly of a single-arm barcode, and
Figure 4B shows the modular assembly of a twin arm barcode;
Figure 5A illustrates a direct nanopore-based detection method according to this invention with a single-arm identifier polymer,
Figure 5B illustrates a direct nanopore-based detection method according to this invention with a twin arm identifier polymer,
Figure 5C illustrates an indirect nanopore-based detection method according to this invention, and
Figure 5D illustrates a multiplexed indirect nanopore-based detection method according to this invention;
Figure 6 illustrates schematically different possible embodiments of the single-molecule detection/quantification method according to this invention, wherein
Figure 6A illustrates an assay method according to this invention in which a twin arm barcode molecular probe is initially immobilised to a solid support,
Figure 6B illustrates an assay method according to this invention in which a twin arm barcode molecular probe is initially free and subseqently captured by a solid support using biotin/streptavidin,
Figure 6C illustrates an assay method according to this invention in which a twin arm barcode molecular probe is initially immobilsed on a first support and subsequently released and recaptured on a second solid support using biotin/streptavidin,
Figure 6D illustrates an assay method according to this invention in which a single arm barcode molecular probe is initially immobilsed on a solid support,
Figure 6E illustrates an assay method according to this invention in which a single arm barcode molecular probe is initially free and captured by a solid support using biotin/streptavidin,
Figure 6F illustrates an assay method according to this invention with a single arm barcode molecular probe, which corresponds to the second target analyte binding molecule in a sandwich-type ELISA setup for binding of the target analyte,
Figure 6G illustrates an assay method according to this invention with a single arm barcode molecular probe with a sandwich-type ELISA setup for binding the target analyte, in which the separated single arm barcode polymer is captured by a second solid support using biotin/ streptavidin before translocation through the nanopore,
Figure 6H illustrates an assay method according to this invention with a single arm barcode molecular probe is immobilised on a first solid substrate for binding of the target analyte and subsequently released to bind a second target analyte binding molecule immobilised on a second solid support,
Figure 6I illustrates the assay method of Figure 6H, in which the single arm barcode molecular probe probe is initially free and then captured by the first solid support using biotin/streptavidin,
Figure 6J illustrates an assay method according to this invention using two different single arm barcode molecular probes directed to different regions of the target analyte, for example different epitopes of the same antigen, and three different solid supports,
Figure 6K illustrates another assay method according to this invention using two different single arm barcode molecular probes directed to different regions of the target analyte, and two different solid supports.

### Examples of embodiments of the present invention

The invention relates to single molecule resolution method of target analyte, preferably protein or peptide, detection and quantification with the nanopore sensor, preferably a solid-state nanopore sensor.

The assay method uses molecular probes comprising of an analyte binding module, i.e. the binding molecule, which is conjugated to a unique structurally modified barcode molecule, i.e. the identifier polymer, which serves as unique digital ID.

In the embodiments described hereinafter the identifier biopolymer is a nucleic acid polymer, for example a DNA molecule. For ease of description, it will be referred to a DNA barcode in the following. The provided examples can however also be performed using different types of XNA molecules (e.g. PNA). The invention described herein can also use alternative identifier biopolymers with a defined unique nanostructure serving as unique barcode identifier.

The barcode, in the examples shown the DNA barcode, is characterized with at least single coding module containing unique structural element or combination of multiple coding modules, each containing unique structural element. The coding module contains single unique, structural element located in the middle of ssDNA or dsDNA molecule. The nucleic acid sequence of the barcodes is used as a nanostructured engineering material rather than a carrier of information which is encoded by primary sequence of DNA.

The invention describes the method where targeted proteins bound to DNA barcoded molecular probes are counted by the nanopore sensor allowing estimation of the absolute number of protein molecules in the analyzed sample.

Alternatively, the number of DNA barcodes liberated from the molecular probes, which have been bound to targeted protein molecules, are counted by the nanopore sensor allowing to determine the absolute number of protein molecules in the analyzed sample.

The molecular binding molecule may for example be a single-chain variable fragment (scFv), a camelid (VHH), a small protein binder, such as a monobody, an affibody, an anticalin or a DARPin. The molecular binding molecule may be a nucleic acid-based protein binder, such as a DNA aptamer or an RNA aptamer.

Nucleic acid barcodes may for example comprise 100bases or base pairs (bp) to 10 kilo bases (kb) or kilo base pairs (kbp). Nucleic acid barcodes may for example range in length from 30 nm to 3000nm. Nucleic acid barcodes may have a diameter ranging from 1nm to 50nm. ssDNA barcodes would be an example for the lower end of this range, while tethered nano-molecule would be examples the upper end of this range.

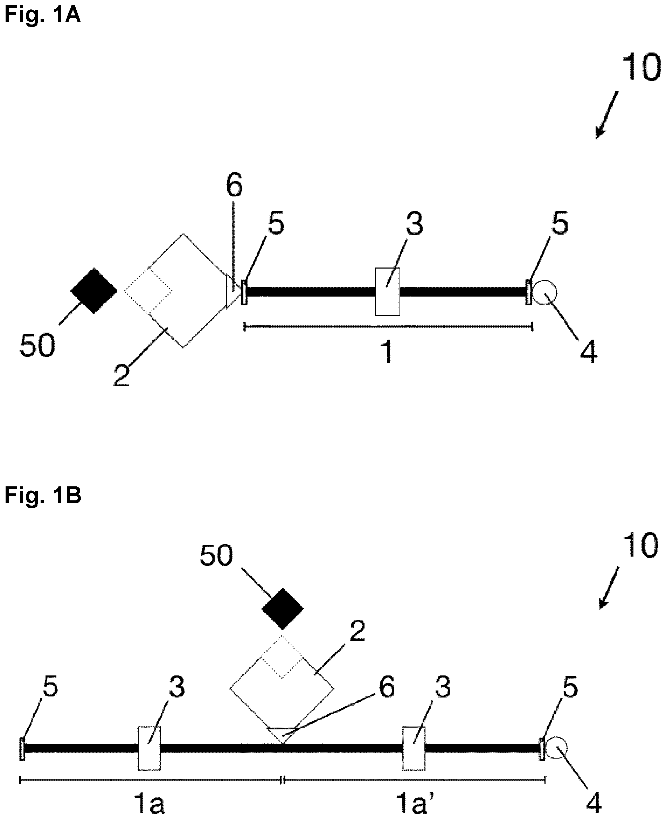

In Figure 1A shows an example of a molecular probe 1 according to this invention, which comprises an antigen binding molecule 2, which is designed to bind an antigen 50 under appropriate conditions, and a single identifier biopolymer1 which may be a DNA barcode. The identifier biopolymer1 contains a structural element 3 which has larger cross-sectional dimensions that the adjacent portions of the identifier biopolymer1. The structural element 3 forms a unique nanostructure which serves to identify the molecular probe 10, respectively the binding molecule 2 comprised in the molecular probe.

The structural element 3 is preferably arranged in a middle portion of the identifier polymer, as shown in Figure 1A.

In embodiments where the identifier biopolymer1 is a DNA barcode, the identifier biopolymer not only provides the unique identification label for the molecular probe 10 but may also function as a polyanionic leader which guides the molecular probe through the pore of a nanopore sensor due to its polyanionic charge. Thereby the nucleic acid identifier 1 ensures the correct orientation of the molecular probe 10 during its translocation. In addition, it ensures an appropriate translocation velocity through the nanopore thus enabling reliable measurements.

The binding molecule 2 may be covalently linked to the identifier biopolymer1 through a conjugation element 6. Possible non-limiting examples for conjugation elements 6 are characterized with thiol group or amine group or azide group or alkynyl group .

The identifier biopolymer1 may comprise cleavable elements 5, such as sequences specific for restriction endonucleases, or UV sensitive photo-cleavable linkers, which enable the separation of the identifier biopolymer1 from the molecular probe 2.

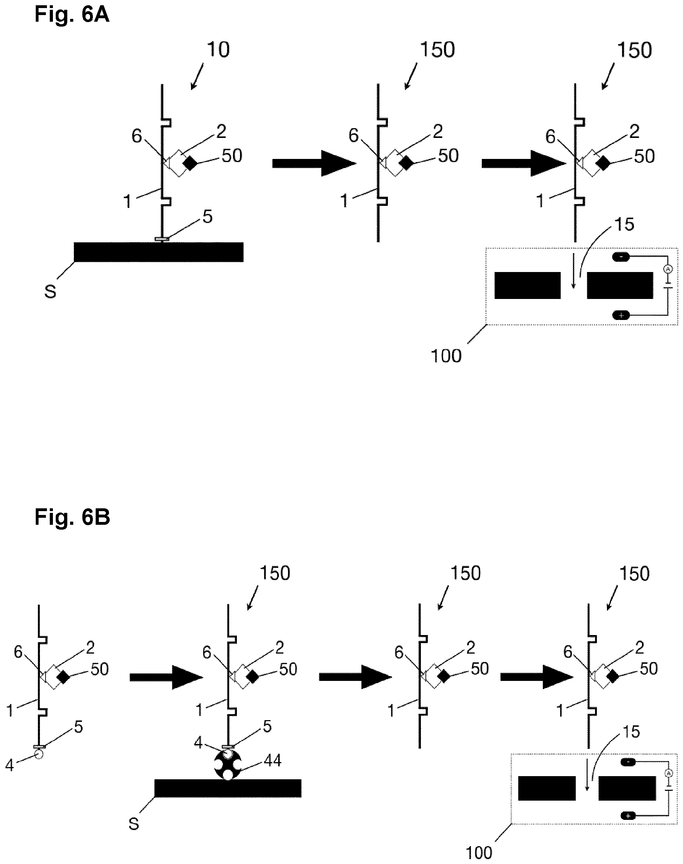

It is also possible to provide the molecular probe with a capture element 4, for example biotin, which may be covalently linked to the free end of the identifier biopolymer1. The capture element 4 serves to non-covalently couple the identifier biopolymer1 or the entire molecular probe 10 to a support via compatible binding element 44 (Figure 6B), such as avidin or streptavidin. A cleavable element 5 may be arranged to allow for separating the identifier biopolymer1, respectively the remainder of the molecular probe 1 from the capture element 4.

The example shown in Figure 1A is a single arm barcode molecular probe, comprising only one identifier polymer 1 conjugated to the binding molecule via one of the ends of the nucleic acid.

Another embodiment of the present invention is presented in Figure 1B which depicts a twin arm barcode molecular probe 10. This molecular probe comprises two identical identifier polymers 1a, 1a' which are liked to each other on one end such that they mirror each other. The binding molecule 2 may be coupled to the twin arm barcode at the junction of the two identifier polymers 1a, 1a', for example using a conjugation element 6. The molecular probe may or may not be coupled to a capture element 4 on a free end of at least one of the identifier biopolymer arms 1a, 1a'.

Providing two identical barcode arms on the same molecular probe serves to bi-directional identification of the same unique signal measured by the nanopore sensor during translocation of the molecular probe.

It is however also possible to design a molecular probe 10 with two non-identical identifier polymers.

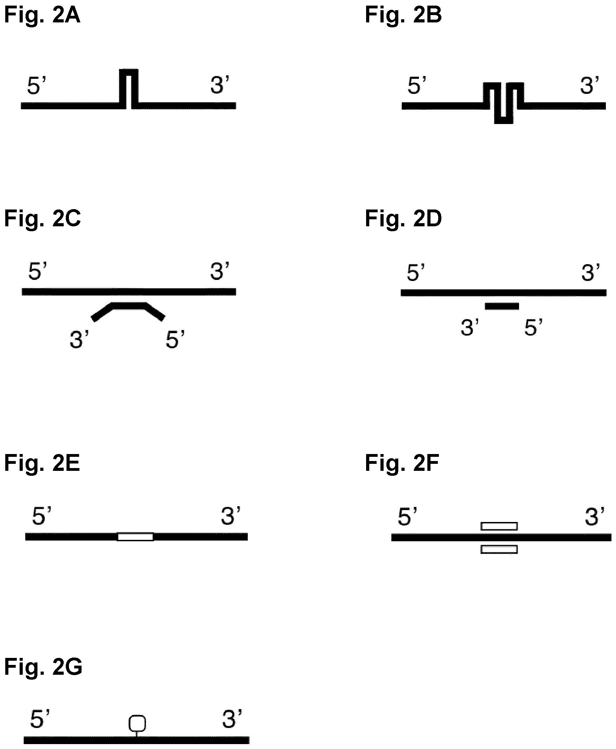

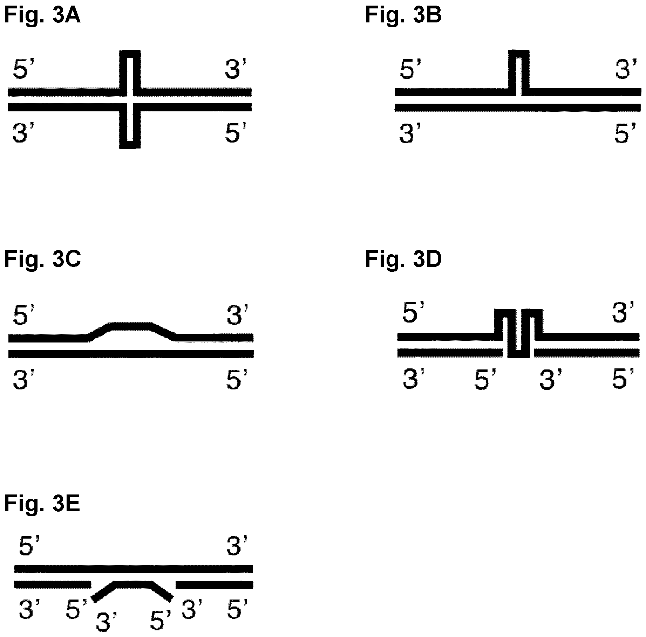

Figures 2A to 2G and Figures 3A to 3J illustrate possible examples of nucleic acid identifier molecules, such as DNA or XNA polymers.

Figure 2A depicts a ssDNA molecule with a hairpin structural element.

Figure 2B depicts a ssDNA molecule with a G4 quadruplex structural element.

Figure 2C depicts a ssDNA molecule with a 3'-5' flap dsDNA patch structural element.

Figure 2D depicts a ssDNA molecule with dsDNA patch structural element.

Figure 2E depicts a ssDNA molecule with ssPNA patch structural element.

Figure 2F depicts a ssDNA molecule with a PNA-DNA-PNA triple helix structural element.

Figure 2G depicts a ssDNA molecule with a nano-molecule tethering structural element.

Figures 3A to 3J are identifier polymers based on a double strand (ds) nucleic acid. The nucleic acid may be DNA or XNA.

Figure 3A depicts a dsDNA molecule with a cruciform structural element formed by two hairpin structures,

Figure 3B depicts a dsDNA molecule with a single hairpin structural element.

Figure 3C depicts a dsDNA molecule with a loop structural element.

Figure 3D depicts a dsDNA molecule with a G4 quadruplex structural element.

Figure 3E depicts a dsDNA molecule with a 3'-5' flap structural element.

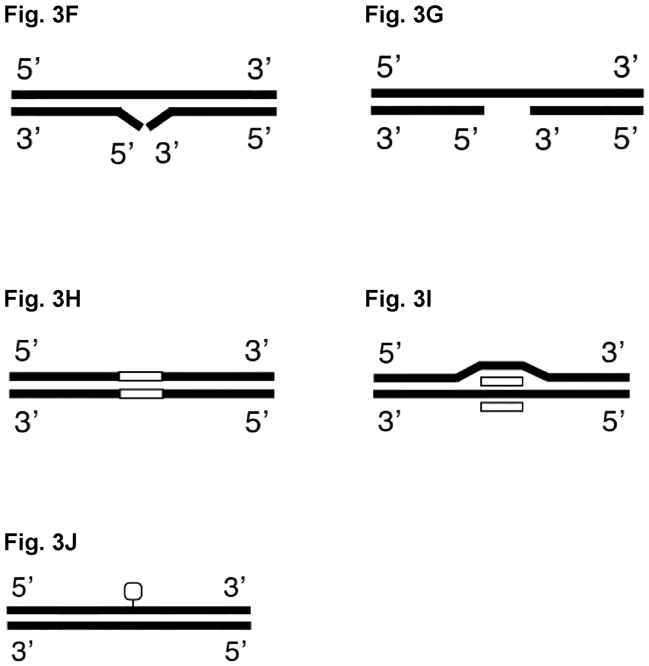

Figure 3F depicts a dsDNA molecule with a 5'-3' flap structural element.

Figure 3G depicts a dsDNA molecule with a gap structural element.

Figure 3H depicts a dsDNA molecule with a dsPNA patch structural element.

Figure 3I depicts a dsDNA molecule with a PNA-DNA-PNA triple helix-loop structural element.

Figure 3J depicts a dsDNA molecule with a nano-molecule tethering structural element.

The invention is however not limited to these exemplary structural elements.

One or more of the structural elements 3 may be present in any identifier biopolymer1.

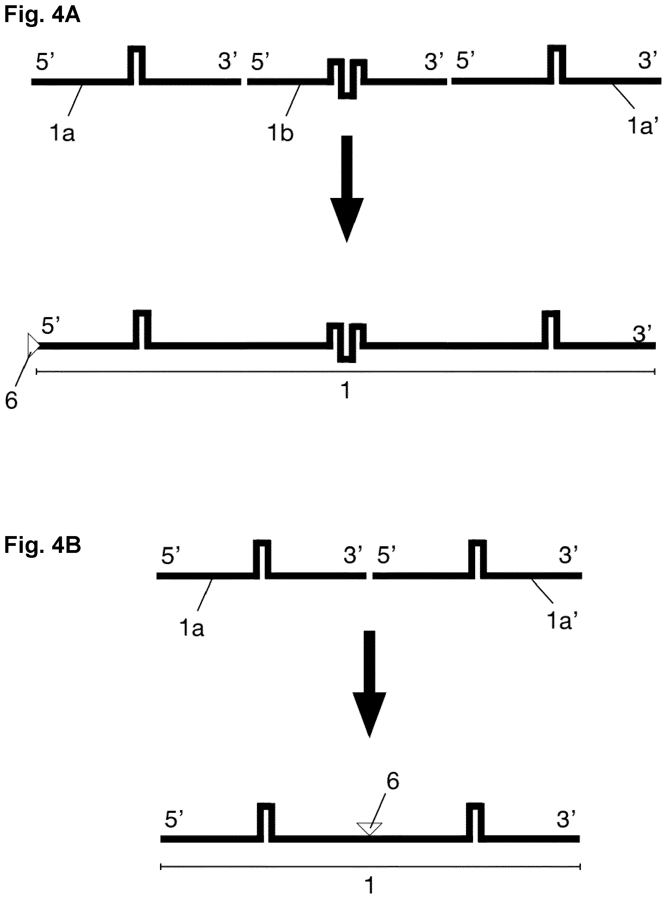

Figure 4 shows a modular assembly of exemplar nucleic barcodes of this invention. Nucleic acid barcodes may be assembled from synthetic building blocks in enzymatic or chemical reactions. The nucleic acid barcodes can be assembled from odd or even number of synthetic building blocks.

An example of an assembly of a single arm DNA barcode is illustrated in Figure 4A. In a first step, two or more ssDNA or dsDNA barcode strands 1a, 1a', 1b are provided. The barcode strands may comprise different structural elements. The ssDNA or dsDNA barcode strands 1a, 1a', 1 b may then be merged, for example in a ligase chain reaction (LCR) or using Click chemistry, resulting in a merged single ssDNA or dsDNA barcode strand 1, which may be conjugated to a binding molecule via conjugation element 6 located at one of the ends. The nucleic acid barcode presents an example of the biopolymer formed by odd number of synthetic building blocks with single unique structural element located in the middle of the barcode. The single unique structural element provides center point of reference within the single arm barcode.

Figure 4B illustrates the assembly of a twin arm ssDNA or dsDNA barcode. In this example two identical ssDNA or dsDNA barcode strands 1a, 1a' are aligned such that the ssDNA barcode strands mirror one another. By joining the aligned strand, for example by LCR or Click chemistry, a single twin arm identifier biopolymer1 is generated. This twin arm identifier biopolymer may be provided with a conjugation element 6 at the junction site of the twin arms. The nucleic acid barcode presents an example of the molecule formed by even number of identical synthetic building blocks with conjugation element located in the middle of the barcode, which may be conjugated to a binding molecule. In such configuration the binding molecule also serves the function of the center point of reference within the twin-arms barcode.

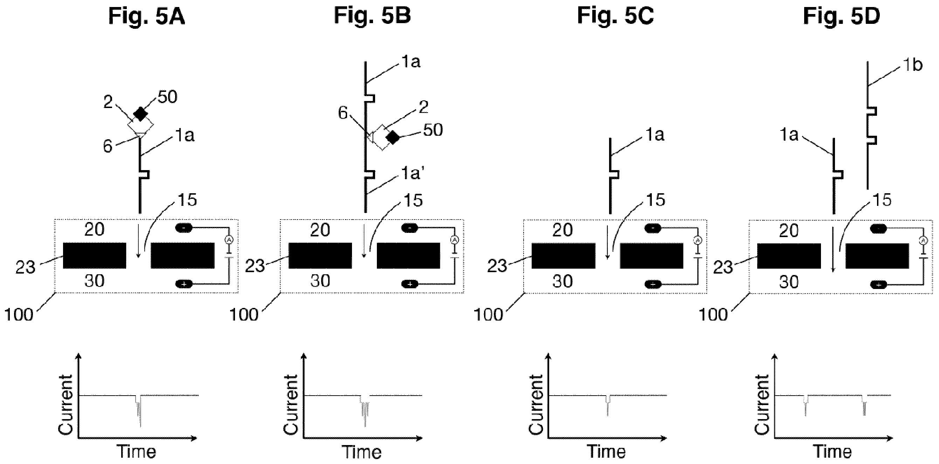

Figures 5A to 5D are schematic illustrations of single-molecule detection and/or quantification assays according to this invention, wherein Figures 5A and 5B illustrate a direct counting method and Figures 5C and 5D illustrate an indirect counting method.

In Figure 5A a single-arm barcode molecular probe 1 with a bound antigen 10 is provided to a nanopore sensor 10 with a nanopore 150 for translocation of the molecular probe 1. The single arm barcode 1 acts as a polyanionic leader guiding the molecular probe towards the nanopore.

The nanopore sensor 100 is preferably a solid-state nanopore sensor using a flow cell with a plurality of nanopores 15 embedded in an electro-resistant membrane 23, whereby each nanopore 15 corresponds to its own electrode. The membrane 23 separates two chambers 20, 30 which are fluidly connected through a nanopore channel 15.

The antigen-molecular probe 1 complex travels from a first chamber 20 comprising an electrolyte and a negative electrode (anode) through nanopore channel 15 into a second chamber 30 with electrolyte and positive electrode (cathode).

Upon translocation of the molecular probe 1 with its unique identifier polymer, i.e. the single-arm barcode, a unique electrical signature is measurable at the nanopore electrode, as is shown in the diagram underneath the nanopore sensor.

When molecular probes with twin arm barcodes 1a, 1a' are passing through a nanopore electrode the obtained signal is more complex as a result of the plurality of structural elements contained in the molecular probe, as shown in Figure 5B, and in particular in the diagram underneath the nanopore sensor of this Figure. Since the twin arm barcode comprises of even number of identical building blocks 1a and 1a' it can translocate via the nanopore sensor bi-directionally giving the same unique electrical signature.

In some assays, it may be useful to detect and/or quantify identifier polymers only, for example after they have been cleaved from the molecular probe. Detection and/or quantification of the separated identifier polymers may provide an indirect counting method for the amount of target analyte in a sample.

In Figure 5C such an indirect counting method is exemplified with single identifier polymer, symbolizing a plurality of identical identifier polymers, detected and/or quantified by a nanopore sensor as described above.

An example for indirect counting of single analyte which is detected by pair of specific molecular probes using sandwich ELISA protocol or detection of two different analytes with single specific molecular probes using sandwich ELISA protocol is shown in Figure 5D. Here a plurality of different identifier polymers 1a, 1b, which have different structural elements, each kind of structural element being correlated with specific binding molecule, are provided to the nanopore sensor. Each unique identifier biopolymer1a, 1b generates a unique identifier signature when passing through the nanopore, as is shown in the diagram underneath the nanopore sensor. Since the barcodes have central location of the structural elements these molecules can translocate via the nanopore sensor bi-directionally giving the same unique electrical signature.

The direct or indirect counting methods of this invention of well adapted for multiplexing, and capable of delivering reliable measurements for highly multiplexed assays, comprising 50 or more, or 100 or more or 200 or different identifier polymers.

In a preferred embodiment the detection and/or quantification assay of this invention may combine the specificity of molecular capture of a traditional immunoassays, for example an ELISA assay, with the single molecule resolution of detection and/or quantification, so called "molecular counting", owed to the ionic current based measurement of a solid-state nanopore sensor.

Different non-limiting exemplary embodiments combining an immunoassay with the nanopore sensor detection are schematically presented in Figures 6A to 6K.

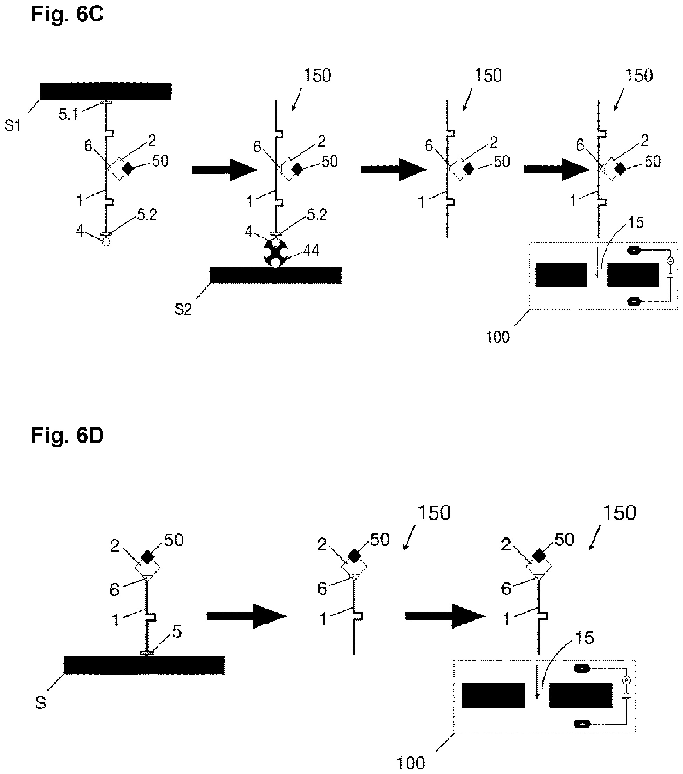

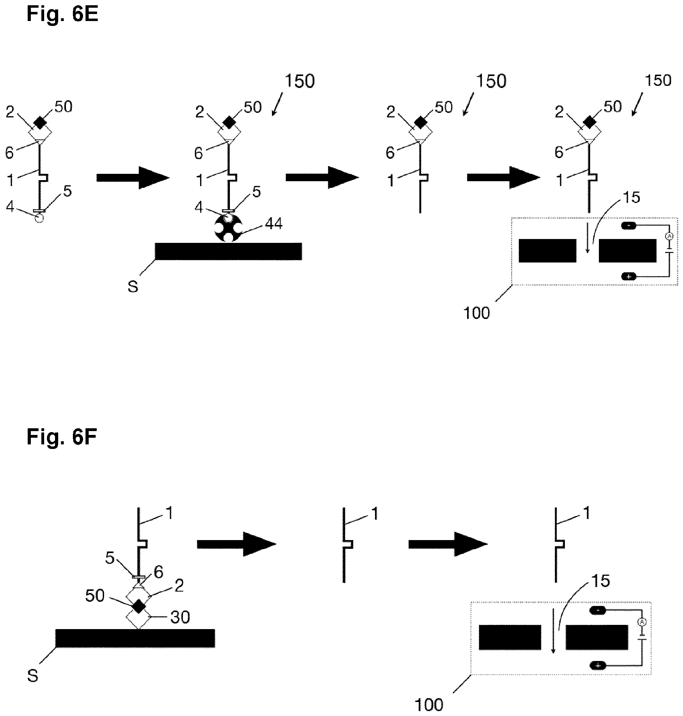

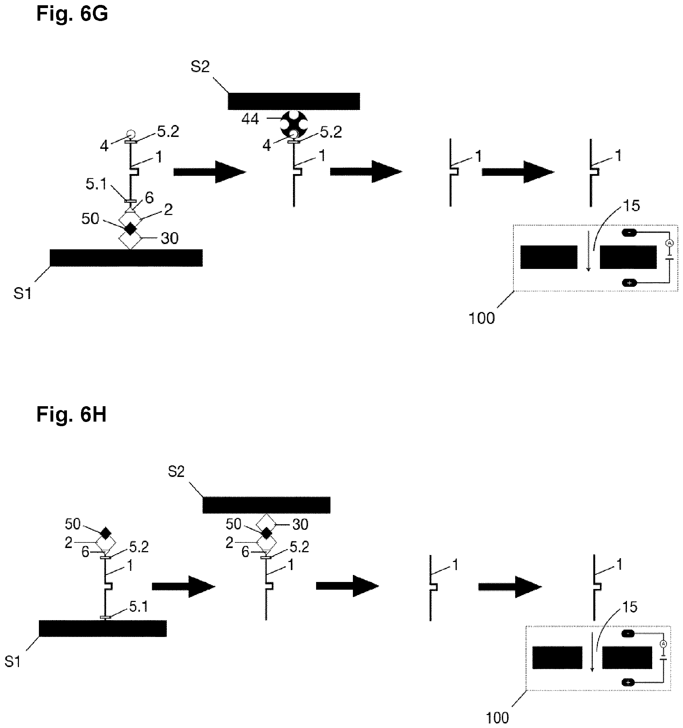

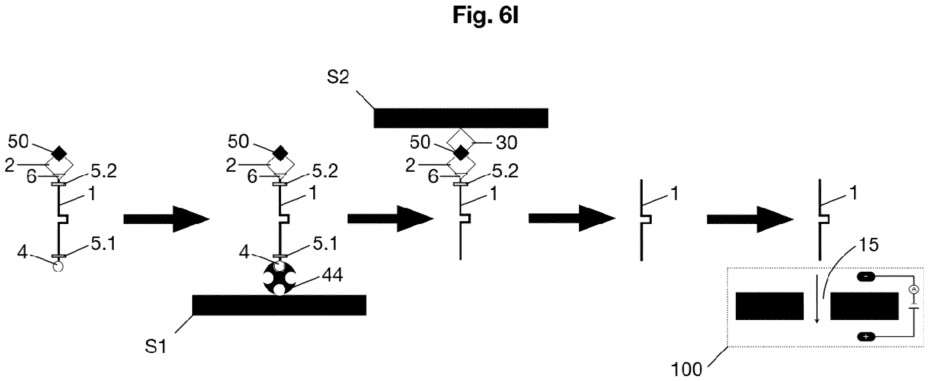

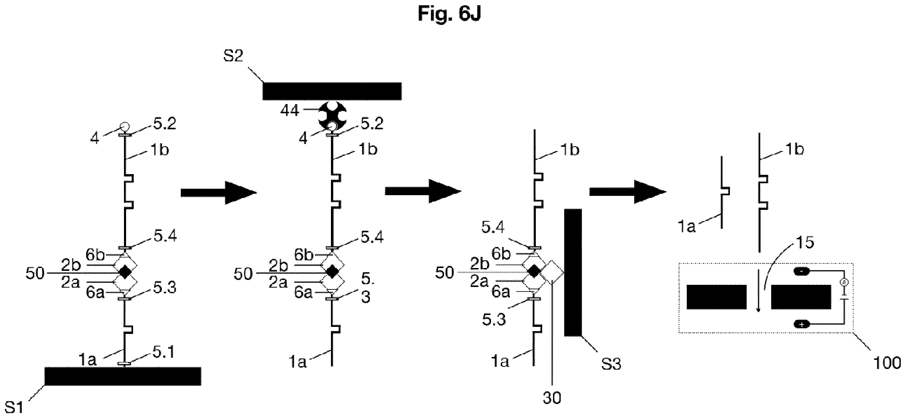

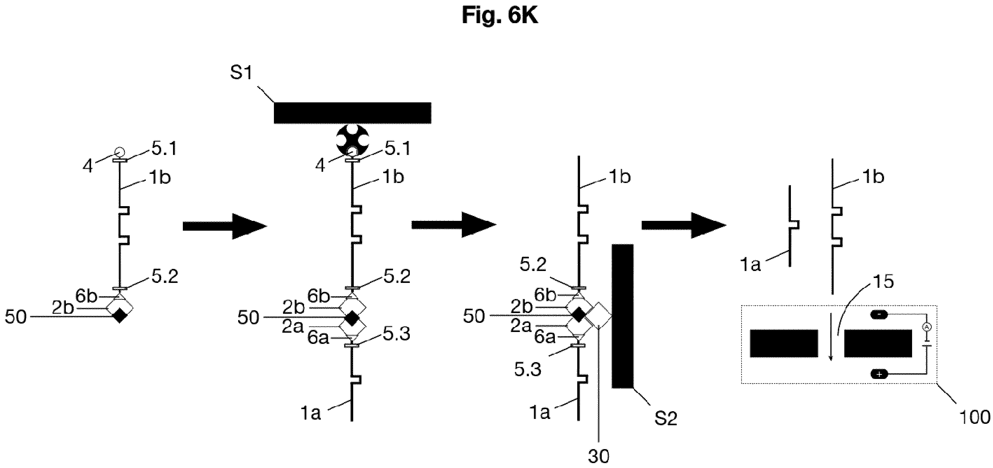

The immunoassay may be performed according to a direct ELISA-type format, as shown in Figures 6A, 6C, and 6D or a sandwich ELISA- tape format as shown in Figure 6F, 6G, 6H and 6J for example. A DNA-carrier probe -like assay is depicted in Figures 6B and 6E, and a hybrid of a DNA carrier and sandwich ELISA is shown in Figures 6I and 6K.

A direct or a sandwich ELISA-type assay may be followed by cleaving the identifier biopolymer off any molecular probes which have bound an antigen and applying these separated identifier polymers to the nanopore sensor as shown in Figures 6F, 6G, 6H, 6I, 6J, and 6K.

In Figure 6A a direct ELISA immunoassay is performed using a molecular probe 10 with a twin-arm DNA barcode as identifier biopolymer1. The molecular probe 1 is attached to a solid support S through one of the arms of the identifier biopolymer 1. The binding molecule 2 of the molecular probe has bound a target antigen 50.

The molecular probe / antigen complex 150 is removed from the solid support S by means of a cleavable element 5 of the identifier polymer and molecular probes bound to the antigen are detected and quantified.

All of the molecular probes, the unloaded molecular probes as well as the molecular probes loaded with an antigen are removed from the solid support and subsequently measured with the nanopore sensor. The molecular probe loaded with an antigen can be distinguished from the unloaded probes, as the loaded probes have a larger footprint and/or different mobility when measured in by the nanopore sensor. Generally, only the probes with bound antigen are counted. It is however possible to count both, loaded and unloaded molecular probes if the ratio between them is of interest.

Molecular probe / antigen complexes 150 is then applied to a nanopore sensor for detection and/or quantification of the complex in the assay sample. The presence of the antigen and/or the total amount of the antigen in the original sample can then be determined on the basis of the results of this measurement.

Using single capture/release step on a solid support S1 allows for better removal and/or washing of non-specific molecules, thereby lowering the background signal noise.

In the assay depicted in Figure 6B, the twin-arm identifier biopolymer1 is further provided with a capture element 4, for example biotin, designed to cooperate with a corresponding binding element 44, such as avidin or streptavidin, which itself is adhered to a solid support S, thereby attaching the molecular probe 10 to said solid support S.

The subsequent steps are performed as in the assay shown in Figure 6A.

In Figure 6C a sandwich ELISA approach is taken for binding the target antigen. The molecular probe 10 is the first provided on a first solid support S1 and contacted with a sample potentially comprising the target antigen. Once the antigen has bound, the molecular probe/antigen complex 150 are removed from the first solid support through a first cleavable element 5.1 positioned at an end portion of the twin arm identifier biopolymer, which is attached to the first solid support S1.

The second arm of the twin arm identifier biopolymer is conjugated to a capture element 4, for example biotin, at the free end of the arm. This capture element enables binding of the released molecular probe/antigen complex 150 to a second solid support S2 through its interaction with a corresponding binding element 44 attached to the second solid support S2.

Using double capture/release step with two distinct solid supports S1, S2 provides the advantage that non-specific molecules can be removed even better, thereby further lowering the background noise.

The molecular probe /antigen complex 150 may then be removed from the second solid support S2 by cleaving the complex 150 from the capture element 4 at a suitably positioned second cleavable element 5.2. Removed complexes 150 may then be detected or quantified using a nanopore sensor 100.

In Figure 6D the method shown in Figure 6A is performed on a molecular probe comprising a single arm identifier biopolymer1.

In Figure 6E the method shown in Figure 6B is performed on initially free molecular probe comprising a single arm identifier biopolymer1.

Figure 6F illustrates an assay method, in which the identifier biopolymer is separated from the molecular probe/antigen complex prior to the nanopore detection/quantification step. The binding assay principle is based on a sandwich ELISA assay, with a first antigen binding molecule 30 provided attached to a solid support S. The first antigen binding molecule 30 binds the antigen on a first binding region of the antigen. This first complex is then contacted with the molecular probe comprising a second antigen binding molecule which binds to a different second region, or a second epitope, of the antigen, thereby linking the molecular probe to the solid support S. In this embodiment, the identifier biopolymer1 of the molecular probe comprises a cleavable element 5 which is preferably located in proximity to the second molecular binding molecule 2. The identifier biopolymer1 may be separated from the molecular probe by cleaving it off at the cleaving element 5.

The so released identifier biopolymer1 may then be detected and/or quantified with a nanopore sensor.

Figure 6G presents a binding assay which is based on a sandwich ELISA format combined with a detection of an identifier molecule which has been released from the molecular probe/antigen complex 150. In this assay, the identifier biopolymer comprises two cleavable elements 5.1 and 5.2, the first cleavable element being arranged for separating the identifier biopolymer1 from the second antigen binding molecule 2, once this has bound the antigen 50, which is coupled to the surface of a first solid support 1 through a first antigen binding molecule 30.

The second cleavable element 5.2 is suitable positioned to separate the identifier biopolymer1 form a second solid support S2 to which it is bound after it has been released from the molecular probe / antigen complex. The identifier molecule may be provided with a capture element 4 for cooperating with a corresponding binding element 44 provided on the surface of said second solid support S2, such as to attach the identifier biopolymer1 to said second solid support S2.

Once released form the second solid support S2 the free identifier biopolymer1 may be detected and/or quantified using a nanopore sensor.

Figure 6H presents a similar sandwich ELISA based binding assay using a first and a second solid support S1, S2 and measuring the released identifier biopolymer1 with the nanopore sensor. In this embodiment using a single arm identifier biopolymer1, the molecular probe is bound via cleavable linker to a first solid support S1 directly through the identifier biopolymer1. After binding of the antigen 50 the antigen/molecular probe complex is removed from the first solid substrate using the first cleavable element 5.1 of the identifier biopolymer1. The released complex is then bound to a second solid support by means of a second antigen binding molecule 30 which binds to a second region, or a second epitope, of the antigen 50, and which is attached to the second solid support S2. A second cleavable element 5.2 suitable positioned on the identifier biopolymer1 may then be used to separate the identifier biopolymer1 from the molecular probe / antigen complex which it bound to the second solid surface S2. The released identifier biopolymer1 is subsequently applied to a nanopore sensor for measurement.

Figure 6I is in principle the same assay as presented in Figure 6H, except that the free molecular probe is used to capture the antigen and subsequently the resulting complex is attached to the surface through a capture element 4, for example biotin, which is coupled to the free end of the identifier biopolymer1 and which cooperates with the corresponding binding element 44 provided on the surface of the first solid support S1.

In Figure 6J a first molecular probe is attached to a first solid support S1 through its identifier biopolymeria. The antigen binding molecule 2a of the molecular probe is arranged for binding a specific region of a target antigen 30. Once bound, the same antigen 30 is then contacted by a second molecular probe having a second binding module 2b, which is specific for a different region of the antigen 30, such that the first binding molecule 2a and the second binding molecule 2b can bind to the antigen 30 at the same time.

The identifier biopolymer1a of the first molecular probe comprises a cleavable element 5.1, for example a restriction site, allowing for removal of the first molecular probe, respectively a complex comprising first molecular probe / antigen 30/ second molecular probe from the first solid support S1.

A subsequent step involves the attachment of the molecular probe / antigen 30/ second molecular probe complex to a second solid support S2. For this purpose, the free end of the second identifier biopolymer1b may be coupled to a capture element 4, for example biotin, such that it can be linked to a corresponding binding element 44, such as avidin or streptavidin, the corresponding binding element 44 being fixed to the solid support S2.

In an additional step, the molecular probe / antigen 30/ second molecular probe complex can be separated from the solid support by separating the complex from the capture element 4, for example by means of another suitably positioned cleavable element 5.2.

A further region of the antigen 30, which is different from the regions bound by the first and the second binding molecules 2a, 2b, may then be bound by a third binding molecule 30 which itself is fixed on a third solid support S3. Consequently, the molecular probe / antigen 30/ second molecular probe complex is bound to a further solid support S3 through another antigen-specific binding interaction. As this assay relies on three different specifically recognized regions, or epitopes, of the antigen, the identification of a specific antigen is extremely reliable. Non-specific binding of antigens which could result in false positive results, is thereby effectively completely avoided.

The identifier polymers 1a, 1b are separated from the molecular probe / antigen 30/ second molecular probe complex attached to the third solid support S3, for example by using further suitably positioned cleavable elements 5.3, 5.4. The released identifier polymers 1a, 1b are then read in a nanopore sensor assay. In doing so, it can be confirmed that both targeted binding regions were present in the bound antigen providing information about the number of intact analyte molecules detected.

By simple measurement of pairs of 1a, 1b polymers more accurate counting of molecules of the single targeted analyte present is accomplished. The current nucleic acid-immuno assays depends on the DNA polymerase and DNA ligase activities (e.g proximity extension assay, proximity ligation assay, NULISA) to establish more accurate measurement of the intact target analytes being bound by two specific probes operating in the sandwich ELISA configuration. The reliance on the DNA polymerase and DNA ligase activities is clear disadvantage of current methods as DNA ligase and DNA polymerase activity can be greatly inhibited by high-salt and heparin present in biological fluids (e.g. urine and blood serum). In our approach, more accurate quantification of the intact analyte molecules can be realized in DNA ligase and DNA polymerase independent manner presenting clear advantage to the current nucleic-acid-immunoassays.

Using triple capture/release step based on three separate solid supports S1, S2, S3 further enhances the effects of the double capture/release method described above. The method allows for maximal effectiveness of washing off non-specific molecules. Due to the tree specific binding interactions with the target antigen, false positive detections of the target antigen are extremely unlikely.

A similar approach involving three binding molecules 2a, 2b and 30, which are each directed to different regions, or epitopes, of the antigen 30, is shown in Figure 6K. In this approach, the first binding of the antigen 30 to the first molecular probe comprising the first binding molecule2a and the first identifier biopolymeria occurs in a liquid reaction mixture, and not on a solid support. The resulting first molecular probe / antigen 30 complex is then attached to a first solid support S1, for example by means of a coupled capture element 4 which coupled to a corresponding binding element 44 fixed on the solid support S1. While immobilized on the solid support S1 through the first molecular probe the antigen 30 can then bind to a second binding molecule 2b comprised in a second molecular probe. This second binding molecule 2b targets a different region from the first binding molecule 2a, such that both binding molecules 2a, 2b can bind the antigen 30 at the same time. In analogy to the procedure described above for Figure 6J, once separated from the solid support the first molecular probe / antigen 30/ second molecular probe complex can now be recruited to a further solid support S2 through a third binding protein 30 fixed in this support S2.

Once the first molecular probe / antigen 30/ second molecular probe complex is bound to the second solid support S2, identifier molecules 1a and 1b are separated from their respective molecular probes and applied to the nanoprobe sensor for measurement.

The different embodiments presented here provide evidence for the versatility of this assay system combining the advantages of immunoassays and with the nanopore sensor sensitivity. It is clear that further variations of this assay system may be considered, and the presented examples are by no means exhaustive.

A particular further advantage of the system and method of this invention is the use of barcode nanostructures as opposed to barcode primary sequences for the identification of specific target analytes. The structural barcodes, or identifier polymers, can be easily read and requires no amplification step which is generally susceptible to alter the result. Therefore, the measurements, and in particular quantitative measurements, are more robust and reliable that conventional counting system using barcode sequences. In addition, a costly and time-consuming sequencing step of the barcode sequence is avoided by providing structural barcode identifiers, which renders the claimed method and system rapid and more efficient compared to conventional identification methods.

Moreover, the modular characteristic of the structural barcodes, i.e. the identifier polymers, allows for coding of a vast information by providing different combinations of either identifier polymers1, or of structural elements 3 present in an identifier biopolymer1. This modularity of the system opens the door for further coding possibilities using the molecular probes of this invention, which can be read using nanopore sensors and subsequently decoded.

## Claims

1. A molecular probe comprising an identifier biopolymer conjugated with a binding molecule able to bind to a target analyte, wherein the identifier biopolymer comprises one or more structural elements having a defined nanostructure, said nanostructure being defined by the primary sequence of the identifier biopolymer, wherein said structural element is configured to generate a unique identification signal when translocating through a nanopore sensor.

2. The molecular probe of claim 1, wherein the identifier biopolymer comprises a polynucleotide molecule selected from a group consisting of a single strand desoxyribonucleic acid (DNA), a single strand DNA, a double strand DNA, a single strand xeno nucleic acid (XNA), a double strand XNA, for example a peptide nucleic acid (PNA), or a combination thereof.

3. The molecular probe of claim 1 or 2, wherein the one or more structural element are formed by a central portion of the identifier biopolymer.

4. The molecular probe of any of claims 1 to 3, wherein the identifier biopolymer is mirror-symmetrical to the transverse median plane of the biopolymer, for example based on a palindromic sequence.

5. The molecular probe of any of claims 1 to 4, wherein the largest cross-sectional dimension of the structural element ranges from 0.5nm to 180nm, or from 1nm to 90nm, 1.5nm to 45nm, or 2nm to 30nm.

6. The molecular probe of any of claims 1 to 5, wherein a terminal portion of a free end of the identifier biopolymer is negatively charged.

7. The molecular probe of any of claims 1 to 6, wherein the identifier biopolymer is coupled to a small molecule, such as biotin or digoxigenin or a fluorescent dye.

8. The molecular probe of any of claims 1 to 7, comprising a single identifier biopolymer attached to one end of the binding molecule, or comprising two identical identifier biopolymers.

9. The molecular probe of any of claims 1 to 8, wherein the size of the binding molecule ranges from 1 to 100kDa, or from 2 to 80kDa or preferably from 5 to 60kDa.

10. The molecular probe of any of claims 1 to 9, wherein the binding molecule is selected from a group consisting of single-chain variable fragment (scFvs), a camelid (VHH), a small protein binder, such as a monobodies, an affibody, an anticalin, a DARPin, or a nucleic acid-based protein binder, such as a DNA aptamer or an RNA aptamer.

11. A method for the detecting and/or quantifying a target analyte in a sample, the method comprising
(i) Providing a molecular probe comprising a binding molecule having specific binding complement to a target analyte, said binding molecule being conjugated with one or more identifier biopolymers having a nanostructure defined by the sequence of said identifier biopolymer, wherein the identifier biopolymer is configured to generate a unique identification signal when translocating through a nanopore sensor,
*(ii)* Incubating the molecular probe with a sample potentially comprising a target analyte for a period of time, such that one or more target analytes are able to bind to the binding molecule comprised in the molecular probe,
(iii)removing any unbound target analytes,
(iv)optionally, removing any molecular probe which has not bound to a target analyte during the incubation period,
(v) optionally, separating the binding molecule from the molecular probe, such as to obtain the identifier biopolymer,
(vi)translocating the molecular probe or, if the binding molecule-target analyte complex has been removed from the molecular probe, the identifier biopolymer through a nanopore sensor to obtain at least one unique identification signal, and
*(vii)* detecting the presence of one or more target analytes in the sample by correlating the obtained a least one unique identification signal to the one or more target analytes.

12. The method of claim 11, wherein two different molecular probes, each being capable of binding different portions of the same target analyte and each comprising a unique identifier biopolymer, are provided such as to detect and/or quantify the target analyte.

13. The method of claim 11, wherein at least two different molecular probes, each being capable of binding a different target analyte and each comprising a unique identifier biopolymer, are provided such as to detect and/or quantify at least two different target analytes.

14. The method of claim 11 to 13, wherein the nanopore sensor is a solid-state nanopore sensor.

15. The method of claim 14, wherein the molecular probe is immobilised on a solid support during the incubation step and wherein either the molecular probe is removed from the solid support or the identifier biopolymer is separated from the molecular probe for translocation though the nanopore sensor.

16. A system for detecting and/or quantifying a target analyte in a sample, the system comprising:
- a molecular probe comprising a binding molecule having specific binding complement to a target analyte, said binding molecule being conjugated with one or more identifier biopolymers having a nanostructure defined by the sequence of said identifier biopolymer, wherein the identifier biopolymer is configured to generate a unique identification signal when translocating through a nanopore sensor, and
- a nanopore sensor comprising a nanopore, said nanopore sensor being configured to obtain a unique identification signal when the molecular probe and/or the identifier biopolymer translocates through the nanopore of the nanopore sensor.
